(19) Europäisches Patentamt
European Patent Office
Office européen des brevets

(11) **EP 4 560 160 A1**

(12) **EUROPEAN PATENT APPLICATION**
published in accordance with Art. 153(4) EPC

(43) Date of publication:
**28.05.2025 Bulletin 2025/22**

(21) Application number: **23842945.0**

(22) Date of filing: **14.07.2023**

(51) International Patent Classification (IPC):
*F16C 19/04* (2006.01)     *F16C 19/52* (2006.01)
*F16C 41/00* (2006.01)     *G01M 13/04* (2019.01)

(52) Cooperative Patent Classification (CPC):
**F16C 19/04; F16C 19/52; F16C 41/00; G01M 13/04**

(86) International application number:
**PCT/JP2023/026157**

(87) International publication number:
**WO 2024/019022 (25.01.2024 Gazette 2024/04)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **19.07.2022 JP 2022114947**

(71) Applicant: **NSK Ltd.**
**Tokyo 141-8560 (JP)**

(72) Inventors:
• **IWASE Shunsuke**
**Fujisawa-shi, Kanagawa 251-8501 (JP)**
• **KOSUGI Daichi**
**Fujisawa-shi, Kanagawa 251-8501 (JP)**
• **MARUYAMA Taisuke**
**Fujisawa-shi, Kanagawa 251-8501 (JP)**

(74) Representative: **Grünecker Patent- und
Rechtsanwälte
PartG mbB
Leopoldstraße 4
80802 München (DE)**

(54) **WATER INTRUSION DETECTION METHOD, WATER INTRUSION DETECTION DEVICE, AND PROGRAM FOR DEVICE USING LUBRICANT**

(57)     A water intrusion detection method for detecting intrusion of water into a lubricant in a device in which a plurality of portions are lubricated using the lubricant, includes: measuring an impedance of an electric circuit including the plurality of portions by applying an AC voltage to the electric circuit while sweeping a frequency; and detecting the intrusion of water into the lubricant by comparing characteristics indicated by a real part and an imaginary part of an impedance measured in a state in which water is not mixed into the lubricant with characteristics indicated by a real part and an imaginary part of the impedance measured by the measurement.

*FIG. 8*

**Description**

TECHNICAL FIELD

The present invention relates to a water intrusion detection method, a water intrusion detection device, and a program for a device using a lubricant.

BACKGROUND ART

In the related art, in a bearing device, a configuration for lubricating rotation of the bearing device by using a lubricant (for example, a lubricating oil or a grease) is widely used. On the other hand, a rotating component such as a bearing device is periodically subjected to state diagnosis, so that an abnormality or a state change in the bearing device is detected at an early stage and occurrence of a failure or the like of the rotating component is suppressed.

In a bearing device using a lubricant, it is required to appropriately monitor a state of an oil film in order to stably continuously perform a rotational motion or to detect damage to the device at an early stage. For example, as examples of a visualization method for an oil film, light interferometry, fluorescence, infrared spectroscopy, and the like are known as methods using light. In the above methods, a light transmissive material is required, and it is difficult to visualize the state of the oil film in the bearing device, particularly the lubricant on a friction surface around a rolling element. In contrast, an electrical technique may be applied in order to evaluate the state of the oil film in the bearing device. For example, Patent Literature 1 discloses a method for detecting a thickness of a lubricating oil film and a metal contact ratio in a rolling device.

CITATION LIST

PATENT LITERATURE

Patent Literature 1: JP2019-211317A

SUMMARY OF INVENTION

TECHNICAL PROBLEM

In a bearing device, various substances may be mixed into an oil film from the outside depending on an operating environment or the like. Since such mixing of various substances may affect a state of a lubricating oil or a state of the bearing device, it is necessary to detect the mixing at an appropriate timing. Examples of the substance to be mixed into the oil film include water. When water intrudes into the rolling device and is further mixed with a lubricant, a function required for the lubricant may be affected. Therefore, it is required to appropriately detect intrusion of water into the rolling device.

In view of the above problems, an object of the present invention is to provide a method of detecting intrusion of water into a lubricant in a bearing device according to an electrical technique.

SOLUTION TO PROBLEM

In order to solve the above problems, the present invention has the following configuration. That is, a water intrusion detection method for detecting intrusion of water into a lubricant in a device in which a plurality of portions are lubricated using the lubricant, includes:

a measurement step of measuring an impedance of an electric circuit including the plurality of portions by applying an AC voltage to the electric circuit while sweeping a frequency; and
a detection step of detecting the intrusion of water into the lubricant by comparing characteristics indicated by a real part and an imaginary part of an impedance measured in a state in which water is not mixed into the lubricant with characteristics indicated by a real part and an imaginary part of the impedance measured in the measurement step.

Further, another aspect of the present invention has the following configuration. That is, a water intrusion detection device for detecting intrusion of water into a lubricant in a device in which a plurality of portions are lubricated using the lubricant, includes:

a measurement unit configured to measure an impedance of an electric circuit including the plurality of portions by applying an AC voltage to the electric circuit while sweeping a frequency; and
a detection unit configured to detect the intrusion of water into the lubricant by comparing characteristics indicated by a real part and an imaginary part of an impedance measured in a state in which water is not mixed into the lubricant with characteristics indicated by a real part and an imaginary part of the impedance measured by the measurement unit.

Further, another aspect of the present invention has the following configuration. That is, a program causes a computer to execute:

a measurement step of measuring, in a device in which a plurality of portions are lubricated using a lubricant, an impedance of an electric circuit including the plurality of portions by applying an AC voltage to the electric circuit while sweeping a frequency; and

a detection step of detecting the intrusion of water into the lubricant by comparing characteristics indicated by a real part and an imaginary part of an impedance measured in a state in which water is not mixed into the lubricant with characteristics indicated by a real part and an imaginary part of the impedance measured in the measurement step.

ADVANTAGEOUS EFFECTS OF INVENTION

According to the present invention, it is possible to provide a method of detecting intrusion of water into a lubricant in a bearing device according to an electrical technique.

BRIEF DESCRIPTION OF DRAWINGS

FIG. 1 is a schematic diagram showing an example of a device configuration during diagnosis according to the present invention.
FIG. 2 is a graph showing a physical model of a bearing device according to the present invention.
FIG. 3 is a graph showing a geometric model according to the present invention.
FIG. 4 is a circuit diagram illustrating an equivalent circuit of the bearing device according to the present invention.
FIGS. 5A and 5B are conceptual diagrams illustrating intrusion of water into the bearing device according to the present invention.
FIGS. 6A and 6B are conceptual diagrams illustrating the intrusion of water into the bearing device according to the present invention.
FIGS. 7A and 7B are diagrams illustrating the equivalent circuit and a change during a frequency sweep according to the present invention.
FIG. 8 is a flowchart of a water intrusion detection process according to an embodiment of the present invention.
FIG. 9 is a graph showing an example of a detection result according to the embodiment of the present invention.
FIG. 10 is a graph showing an example of the detection result according to the embodiment of the present invention.
FIG. 11 is a graph showing an example of the detection result according to the embodiment of the present invention.
FIG. 12 is a graph showing an example of the detection result according to the embodiment of the present invention.

DESCRIPTION OF EMBODIMENTS

Hereinafter, embodiments of the present invention will be described with reference to the drawings. The embodiments described below are embodiments for explaining the present invention, and are not intended to be interpreted to limit the present invention, and all the configurations described in the embodiments are not necessarily essential configurations for solving the problem of the present invention. In the drawings, the same components are denoted by the same reference numerals, thereby showing a correspondence relation therebetween.

<First Embodiment>

Hereinafter, a first embodiment of the present invention will be described. In the following description, a ball bearing is described as an example of a rolling bearing included in a bearing device, but the present invention is not limited thereto. The present invention can also be applied to a rolling bearing that is a bearing capable of using a lubricant and has another configuration in which intrusion of water from the outside is assumed. Examples of a type of the rolling bearing to which the present invention may be applied include a deep groove ball bearing, an angular contact ball bearing, a tapered roller bearing, a cylindrical roller bearing, and a self-aligning roller bearing. Further, the present invention can also be applied to other rolling devices, for example, an axle bearing, a drive oil gear device, a cylinder, a spindle, and the like.
In the present description, the term "water" refers to all types of liquids that generate an impact on a lubricant to be described later. Therefore, even in a case in which the term "water" is simply described, the water may be treated as including fresh water, saltwater, muddy water, a snow melting agent, a coolant fluid, an abrasive, a liquid in which an electrolyte is dissolved, and the like.

[Device Configuration]

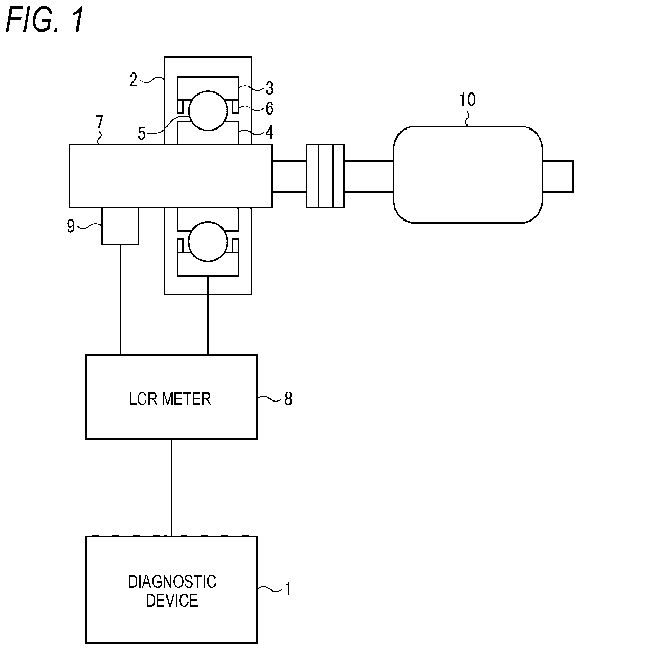

FIG. 1 is a schematic configuration diagram showing an example of an overall configuration when a diagnostic device 1 according to the present embodiment performs diagnosis. In FIG. 1, a bearing device 2 to which a water intrusion detection method according to the present embodiment is applied, and the diagnostic device 1 that performs water intrusion detection and performs the diagnosis are provided. The configuration shown in FIG. 1 is an example, and different configurations may be used according to a configuration of the bearing device 2 and the like. Further, although a configuration in which the bearing device 2 includes one rolling bearing is shown in FIG. 1, the present invention is not limited thereto, and one bearing device 2 may include a plurality of rolling bearings.

In the bearing device 2, the rolling bearing rotatably supports a rotation shaft 7. The rotation shaft 7 is supported by a housing (not shown) that covers the outside of the rotation shaft 7 via the rolling bearing as a rotating component. The rolling bearing includes an outer ring (an outer member) 3 which is a fixed ring internally fitted to the housing, an inner ring (an inner member) 4 which is a rotating ring externally fitted to the rotation shaft 7, a plurality of balls (rollers) which are a plurality of rolling elements 5 arranged between the inner ring 4 and the outer ring 3, and a retainer (not shown) that retains the rolling elements 5 such that the rolling elements 5 can roll freely. Here, although the configuration in which the outer ring 3 is fixed is used, a configuration in which the inner ring 4 is fixed and the outer ring 3 rotates may be used. In addition, a seal 6 that is a peripheral member for preventing entry of dust into the periphery of the rolling elements 5 and leakage of a lubricating oil is provided. Here, the configuration in which the seal 6 is provided is shown, and the intrusion of water may be assumed in the configuration. In the rolling bearing, friction between the inner ring 4 and the rolling elements 5 and friction between the outer ring 3 and the rolling elements 5 are reduced by a predetermined lubrication method. The lubrication method is not particularly limited, and for example, grease lubrication, oil lubrication, or the like is used and supplied to the inside of the rolling bearing. A type of the lubricant is also not particularly limited.

A motor 10 is a motor for driving, and supplies power by rotation to the rotation shaft 7. The rotation shaft 7 is connected to an LCR meter 8 via a rotation connector 9. The rotation connector 9 may be configured with, for example, a carbon brush, and is not limited thereto. Further, the bearing device 2 is also electrically connected to the LCR meter 8, and at this time, the LCR meter 8 also functions as an AC power supply for the bearing device 2.

The diagnostic device 1 operates as a water intrusion detection device capable of executing the detection method according to the present embodiment. At the time of diagnosis, the diagnostic device 1 instructs the LCR meter 8 to use, as inputs, an angular frequency $\omega$ of the AC power supply and an AC voltage V, and acquires, as outputs corresponding thereto, an impedance Z of the bearing device 2 from the LCR meter 8. Then, the diagnostic device 1 detects information related to a state of the lubricant in the bearing device 2 by using these values. Details of the detection method will be described later.

The diagnostic device 1 may be implemented by, for example, an information processing device including a control device, a storage device, and an output device, which are not shown. The control device may include a central processing unit (CPU), a micro processing unit (MPU), a digital single processor (DSP), or a dedicated circuit. The storage device includes volatile and nonvolatile storage media such as a hard disk drive (HDD), a read only memory (ROM), and a random access memory (RAM), and may input and output various kinds of information in response to an instruction from the control device. The output device includes a speaker, a light, or a display device such as a liquid crystal display, and performs notification to an operator in response to the instruction from the control device. A notification method by the output device is not particularly limited, and for example, may be an auditory notification by voice or a visual notification by screen output. The output device may be a network interface having a communication function, and may perform a notification operation by transmitting data to an external device (not shown) via a network (not shown). For example, when water intrusion diagnosis is performed based on a detection result, a notification content is not limited to a notification when the intrusion of water is detected, and may include a notification indicating that the bearing device 2 is normal.

[Physical Model]

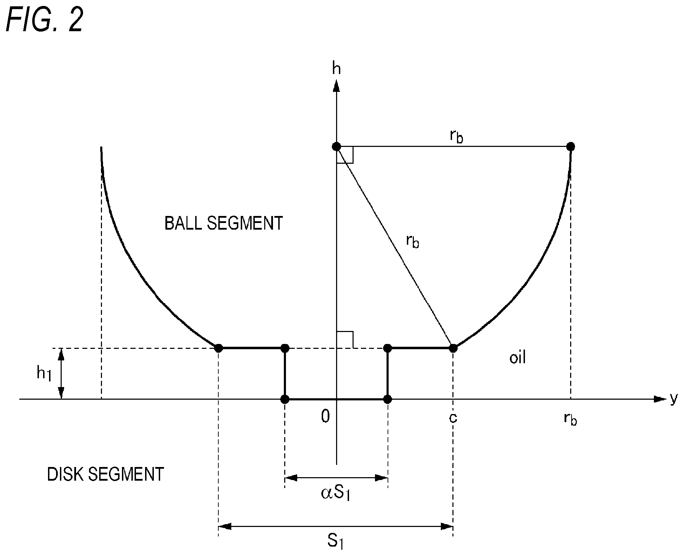

First, a contact state between the rolling elements 5 and the outer ring 3 (or the inner ring 4) in the bearing device 2 will be described with reference to FIG. 2. FIG. 2 is a graph showing a physical model when a ball segment and a disk segment are in contact with each other. The ball segment corresponds to the rolling element, and the disk segment corresponds to the outer ring 3 (or the inner ring 4). An h axis indicates a direction of an oil film thickness, and a y axis indicates a direction orthogonal to the direction of the oil film thickness. Variables shown in FIG. 2 are as follows.

$S_1$: Hertzian contact region
c: Hertzian contact circle radius (= $S_1/2$)

α: breakage rate of oil film (metal contact ratio) $(0 \leq \alpha < 1)$
$r_b$: radius of ball segment
$\alpha S_1$: actual contact region (breakage region of oil film)
h: oil film thickness
$h_1$: oil film thickness in Hertzian contact region

In the Hertzian contact region, a ratio of a range in which a metal is in contact to a range in which the metal is not in contact is $\alpha$: $(1 - \alpha)$. In an ideal state in which the ball segment and the disk segment are not in contact with each other, $\alpha = 0$, and when y = 0, h > 0.

The oil film thickness h shown in FIG. 2 is expressed by the following formulas.

$$h = 0 \ (-\alpha S_1/2 \leq y \leq \alpha S_1/2)$$

$$h = h_1 \ (-c \leq y < -\alpha S_1/2 \text{ or } \alpha S_1/2 < y \leq c)$$

$$h = h_1 + \sqrt{(r_b{}^2 - c^2)} - \sqrt{(r_b{}^2 - y^2)} \ (-r_b \leq y < -c \text{ or } c < y \leq r_b) \ ... \ (1)$$

In an actual rolling bearing, since the rolling elements 5 are elastically deformed when receiving a load, the rolling elements 5 are not strictly spheres, but in the present embodiment, the rolling elements 5 are assumed to be spheres and the above Formula (1) is used. Therefore, a formula used to acquire the oil film thickness is not limited to Formula (1), and other calculation formulas may be used.

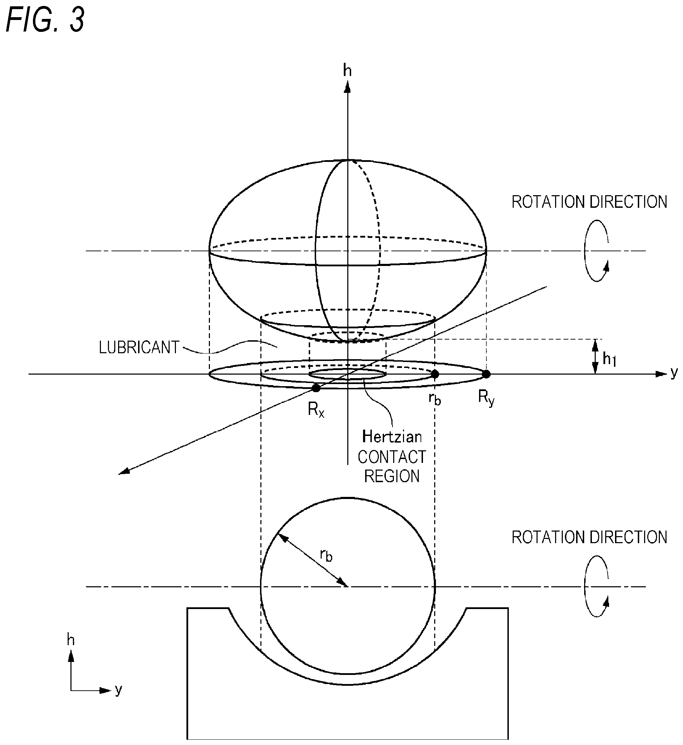

FIG. 3 is a diagram showing a geometric model of the rolling bearing. An x axis indicates an axis direction orthogonal to each of the y axis and the h axis. Variables shown in FIG. 3 are as follows. The same symbols as those in FIG. 2 correspond to the same variables.

$R_x$: effective radius (x axis)
$R_y$: effective radius (y axis)
$h_1$: oil film thickness in Hertzian contact region
$r_b$: radius of ball segment

As shown in FIG. 3, it is assumed that the rolling element 5 rotates about the y axis and a load (an axial load) is applied in a y axis direction.

[Equivalent Electric Circuit]

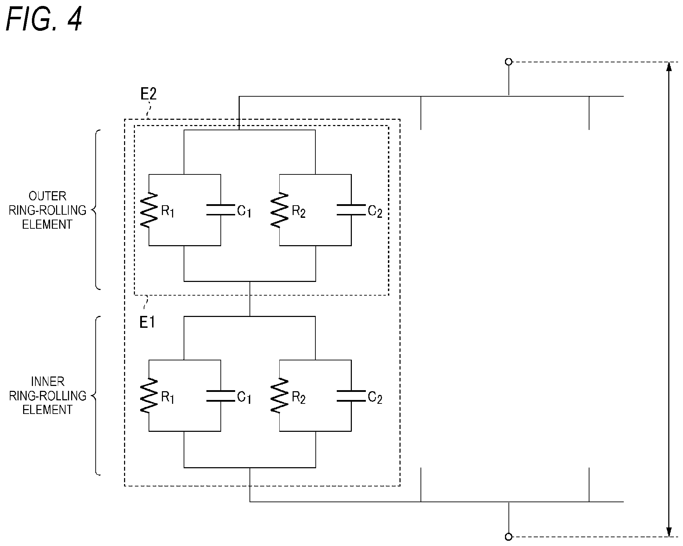

FIG. 4 is a diagram showing the physical model shown in FIG. 2 by an electrically equivalent electric circuit (an equivalent circuit). An equivalent circuit E1 includes a resistor $R_1$, a resistor $R_2$, a capacitor $C_1$, and a capacitor $C_2$. The resistor $R_1$ corresponds to a resistor in the breakage region $(= \alpha S_1)$. The resistor $R_2$ corresponds to a resistor in the vicinity of the breakage region. The capacitor $C_1$ corresponds to a capacitor formed by an oil film in the Hertzian contact region, and has a capacitance $C_1$. The capacitor $C_2$ corresponds to a capacitor formed by an oil film in the vicinity of the Hertzian contact region $(-r_b \leq y < -c$ and $c < y \leq r_b$ in FIG. 2), and has a capacitance $C_2$. The Hertzian contact region $(= S_1)$ forms a parallel circuit of the resistor $R_1$ and the capacitor $C_1$ in the equivalent circuit E1. The vicinity of the Hertizain contact region forms a parallel circuit of the resistor $R_2$ and the capacitor $C_2$ in the equivalent circuit E1. Further, the parallel circuits are connected in parallel to form the equivalent circuit E1. At this time, it is assumed that the lubricant is filled in the vicinity of the Hertzian contact region $(-r_b \leq y < -c$, and $c < y \leq r_b$ in FIG. 2).

An impedance of the equivalent circuit E1 is denoted by Z. Here, an AC voltage V applied to the equivalent circuit E1, a current I flowing through the equivalent circuit E1, and a complex impedance Z of the entire equivalent circuit E1 are expressed by the following Formulas (2) to (4).

$$V = |V|\exp(j\omega t) \ ... \ (2)$$

$$I = |I|\exp(j\omega t - j\theta) \ ... \ (3)$$

$$Z = V/I = |V/I|\exp(j\theta) = |Z|\exp(j\theta) \ ... \ (4)$$

j: imaginary number

ω: angular frequency of AC voltage

t: time

θ: phase angle (shift in phase between voltage and current)

Further, focusing on one rolling element 5, an equivalent circuit E2 is formed between the outer ring 3 and the rolling element 5 and between the inner ring 4 and the rolling element 5. Here, an electric circuit formed by the outer ring 3 and the rolling element 5 is located on an upper side, and an electric circuit formed by the inner ring 4 and the rolling element 5 is located on a lower side, and the locations may be reversed. Around one rolling element 5, these electric circuits are connected in series to form the equivalent circuit E2. Further, since the plurality of rolling elements 5 are provided, the equivalent circuits E2 are connected in parallel, so that an equivalent circuit of the entire rolling bearing as shown in FIG. 4 is configured.

[Intrusion of Water]

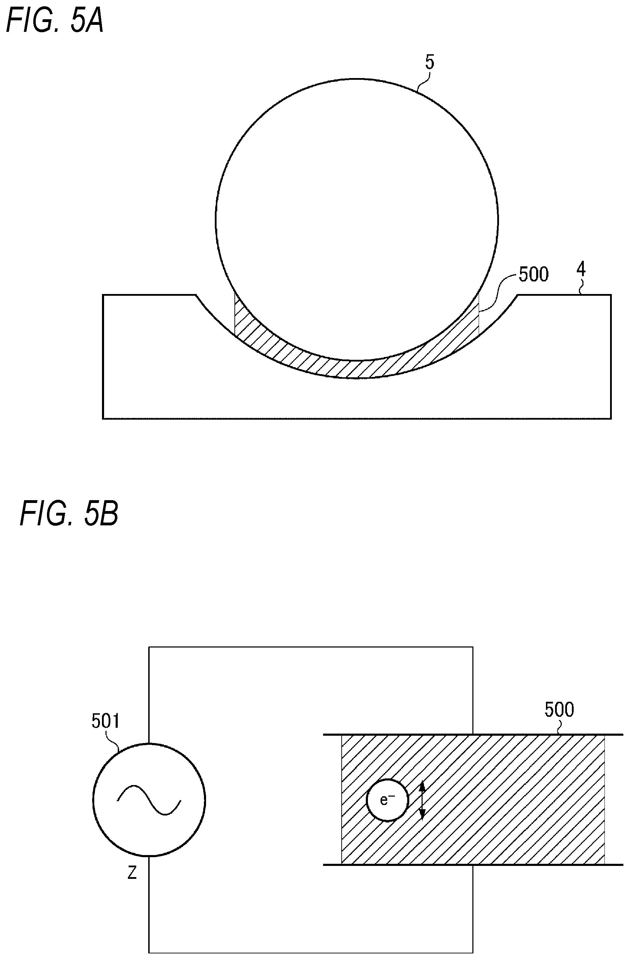

The intrusion of water into the bearing device 2 according to the present embodiment will be described with reference to FIGS. 5A to 6B. FIGS. 5A and 5B are conceptual diagrams illustrating a state in which water does not intrude into the lubricant. In FIG. 5A, a lubricant 500 is filled between an outer ring 4 and the rolling element 5. At this time, as described above, when an AC voltage is applied from an AC power supply 501 to the bearing device 2, the lubricant 500 functions as a capacitor.

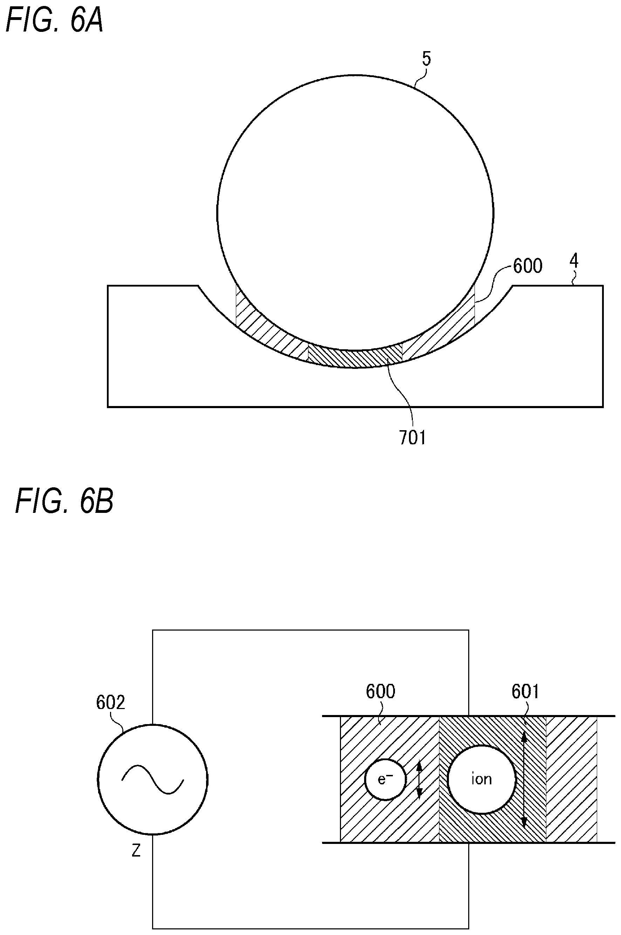

On the other hand, FIGS. 6A and 6B are conceptual diagrams illustrating a state in which water intrudes into the lubricant. In FIG. 6A, a lubricant 600 is filled between the outer ring 4 and the rolling element 5, and water 601 intrudes into a part of the lubricant 600. In this state, when an AC voltage is applied from an AC power supply 602 to the bearing device 2, the lubricant 600 functions as a capacitor, but characteristics different from those in FIGS. 5A and 5B due to the influence of the intruded water 601 are shown. This is because the current is assumed to more easily flow due to the influence of the water 601 intruded into the lubricant 600. In other words, in the state of FIG. 5B in which water does not intrude into the lubricant, the current is less likely to flow than in the state of FIG. 6B.

[Sweep of Frequency of AC power supply]

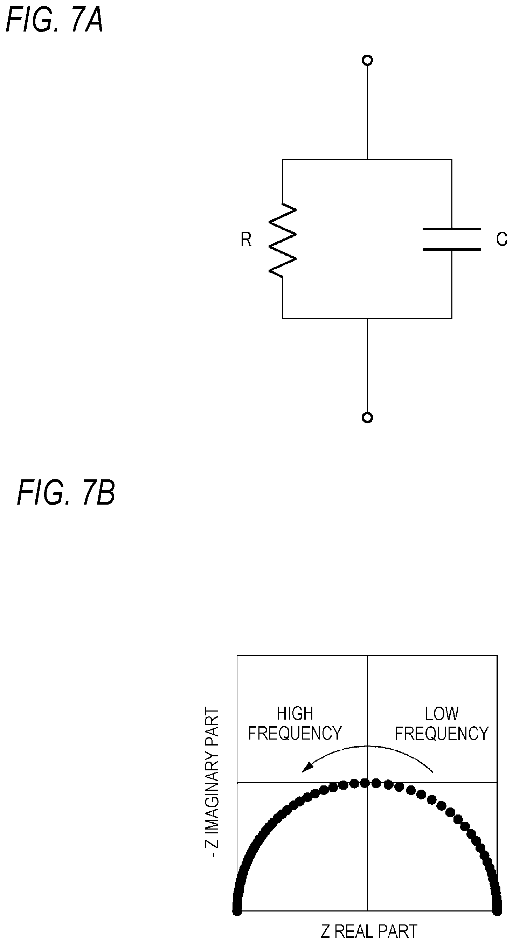

FIGS. 7A and 7B are diagrams illustrating a detection example obtained when a frequency of the AC power supply is swept with respect to the bearing device 2 according to the present embodiment. FIG. 7A shows a parallel circuit of a resistor R and a capacitor C that focuses on a part of the equivalent circuit of the bearing device 2 shown in FIG. 4. FIG. 7B shows, by using a Nyquist diagram, an impedance acquired when the frequency of the AC power supply is swept and applied with respect to the equivalent circuit shown in FIG. 7A. In FIG. 7B, the horizontal axis represents a real part (a Z real part) of the complex impedance Z, and the vertical axis represents an imaginary part (a - Z imaginary part) of the complex impedance Z.

As shown by an arrow in FIG. 7B, in the equivalent circuit around the lubricant, when the frequency of the AC power supply is swept from a low frequency to a high frequency, a change in a semicircular shape is shown. The applicant of the present application specifies the fact that such a change in the characteristics occurs when water intrudes into the lubricant. Specific examples of the change will be described later as test results.

[Processing Flow]

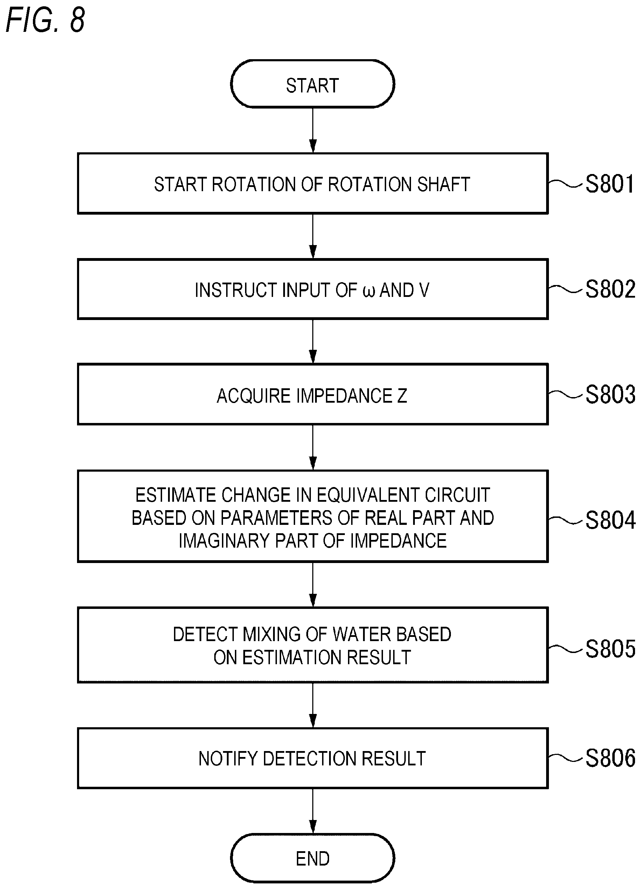

FIG. 8 is a flowchart of a water intrusion detection process according to the present embodiment. The process is executed by the diagnostic device 1, and may be implemented by, for example, reading out a program for implementing the process according to the present embodiment from the storage device (not shown) and executing the program by the control device (not shown) included in the diagnostic device 1.

In step S801, the diagnostic device 1 causes the motor 10 to start rotation of the rotation shaft 7. Accordingly, the inner ring 4 connected to the rotation shaft 7 starts to rotate. The motor 10 may be controlled by a device other than the diagnostic device 1. At this time, the motor 10 may be controlled such that a constant load (for example, the axial load) is applied. The control of applying the load may be performed by a device other than the diagnostic device 1.

In step S802, the diagnostic device 1 controls the LCR meter 8 to supply the AC voltage V of the angular frequency ω to the bearing device 2 while sweeping the frequency by using an AC power supply (not shown) included in the LCR meter 8. Accordingly, the AC voltage V of the angular frequency ω is applied to the bearing device 2.

In step S803, the diagnostic device 1 acquires the impedance Z from the LCR meter 8 as the output meter 8. That is, the LCR meter 8 outputs the impedance Z (the real part, the imaginary part) corresponding to the input in S802. That is, the LCR meter 8 outputs the impedance Z (the real part, the imaginary part) to the diagnostic device 1 as detection results of the bearing device 2 with respect to the AC voltage V and the angular frequency ω of the AC voltage which are inputs.

In S804, the diagnostic device 1 estimates a change in the equivalent circuit based on parameters of the impedance acquired in S803. In a state in which water is not mixed in the lubricant of the bearing device 2, an output result based on the equivalent circuit as shown in FIGS. 3, 7A and 7B is acquired, and this equivalent circuit can be defined in advance. On the other hand, when water intrudes into the lubricant, it is possible to specify deviation based on the output result corresponding to the equivalent circuit. In this step, the change in the equivalent circuit is estimated based on a comparison with a parameter defined in advance in the state in which water does not intrude into the lubricant. The parameter in the state in which water does not intrude into the lubricant may be measured or defined in advance according to the configuration of the bearing device 2, and may be stored in a storage unit or the like. Since the acquired output result changes according to the degree of intrusion of water, the degree of intrusion may also be estimated. Detailed examples of the change in the output result will be described later.

In S805, the diagnostic device 1 detects the intrusion of water based on an estimation result of S804, and performs the state diagnosis. Although a diagnosis content here is not particularly limited, for example, a configuration may be adopted in which a threshold value is set in advance for each parameter and a comparison with the threshold value is performed to diagnose normality or abnormality. In addition, a configuration may be adopted in which a plurality of threshold values may be set in advance according to the urgency of the abnormality, and the urgency may be diagnosed by comparison with the threshold values.

In S806, the diagnostic device 1 notifies a user of a diagnosis result acquired in S805. Although the notification method here is not particularly limited, for example, a configuration may be adopted in which the presence or absence of the intrusion of water and the degree of intrusion of water are displayed on a screen or notified by voice. Then, the processing flow ends.

[Examples of Water Intrusion Detection]

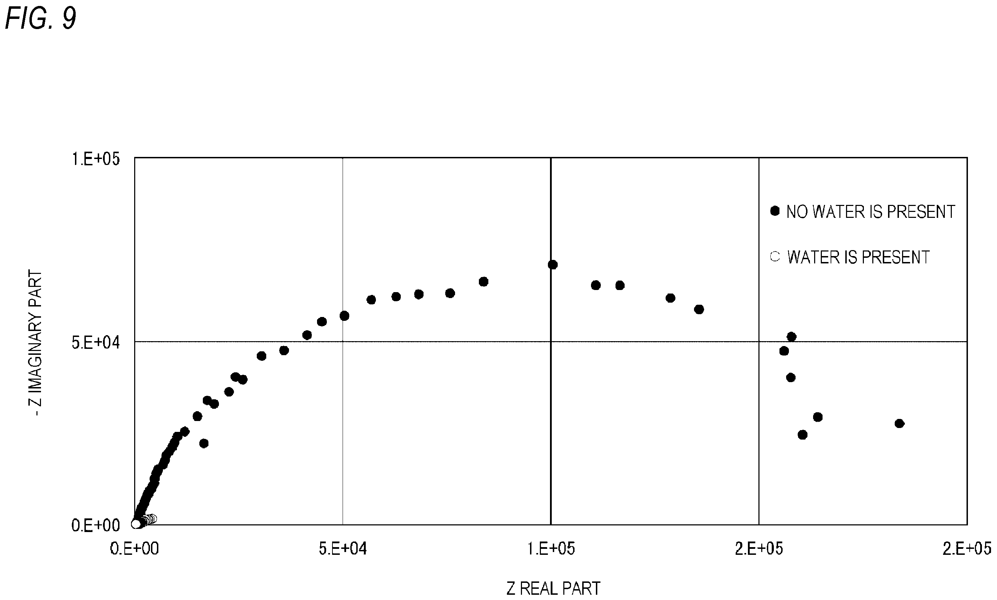

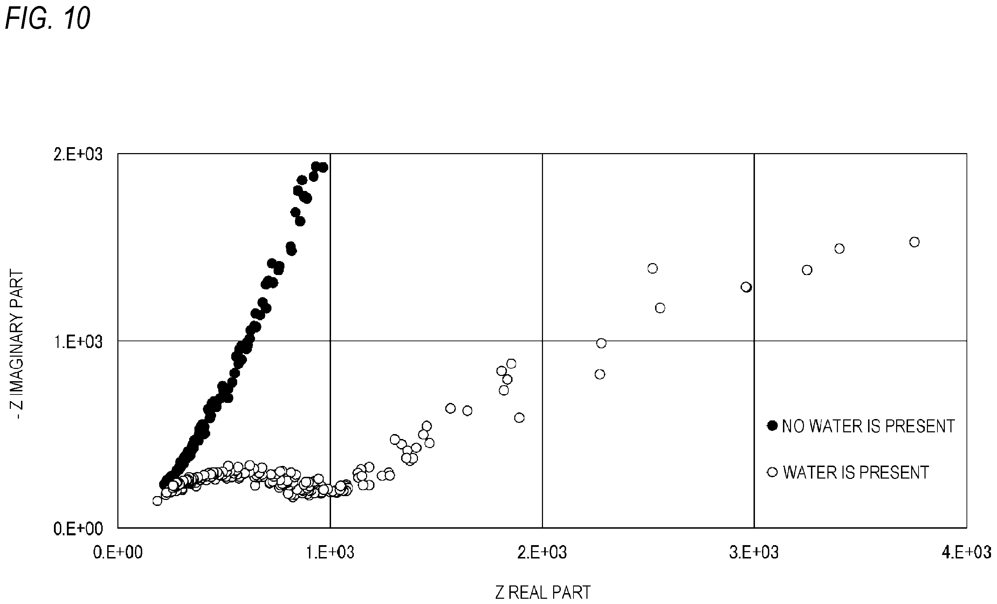

Hereinafter, examples of water intrusion detection according to the present embodiment will be described with reference to FIGS. 9 and 10.

FIG. 9 shows, by using a Nyquist diagram, an example of a detection result acquired while sweeping a frequency (20 Hz to 1 MHz) with respect to the voltage (1 V) of the AC power supply. In FIG. 9, the horizontal axis represents the real part (the Z real part) of the impedance Z, and the vertical axis represents the imaginary part (the - Z imaginary part) of the impedance Z.

Black circles indicate a detection result in a case in which the intrusion of water into the lubricant does not occur. On the other hand, white circles indicate a detection result in a case in which the intrusion of water into the lubricant occurs. In this way, it is possible to identify and specify an apparent difference in the output according to the presence or absence of the intrusion of water.

FIG. 10 is a Nyquist diagram focusing on the lower left portion of FIG. 9 and showing the lower left portion at a different scale. Therefore, test conditions and the like are the same as those in FIG. 9. Also with reference to FIG. 10, a difference in the output result of the impedance acquired according to the presence or absence of the intrusion of water into the lubricant is apparent.

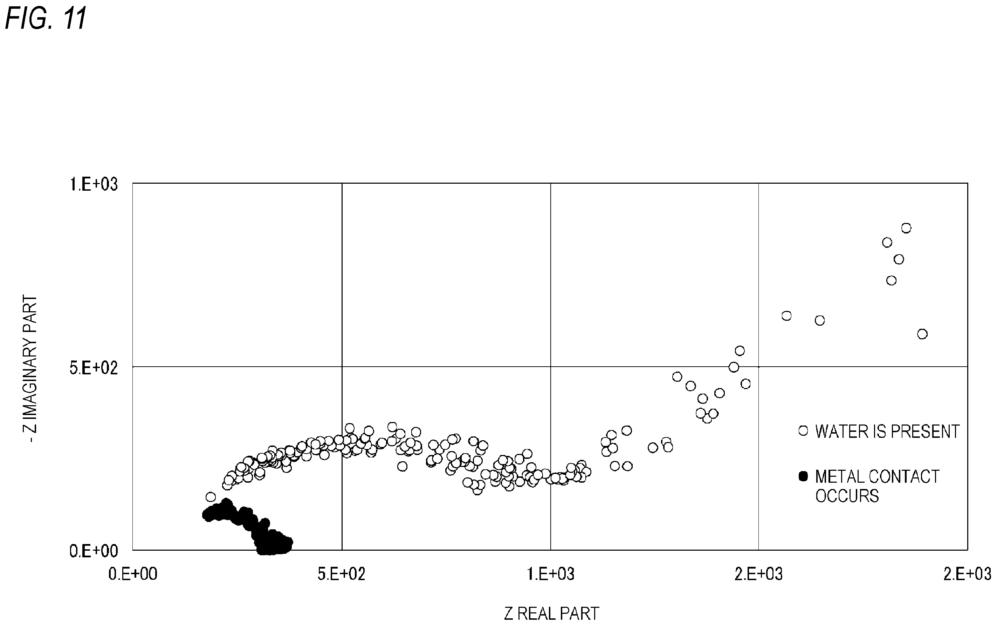

Next, a difference between metal contact that may occur in the configuration of the bearing device as shown in FIG. 3, and the intrusion of water will be described. FIG. 11 shows examples of the detection result in a case in which the metal contact occurs and in the case in which the intrusion of water into the lubricant occurs. The white circles indicate the detection result in the case in which the intrusion of water into the lubricant occurs, and is the same as the example in FIG. 10. On the other hand, the black circles in FIG. 11 show an example of the detection result in a case in which the metal contact occurs between the rolling element and the outer ring (or the inner ring). In this way, it is possible to identify and specify the apparent difference in the output in the case in which the intrusion of water occurs and in the case in which the metal contact occurs.

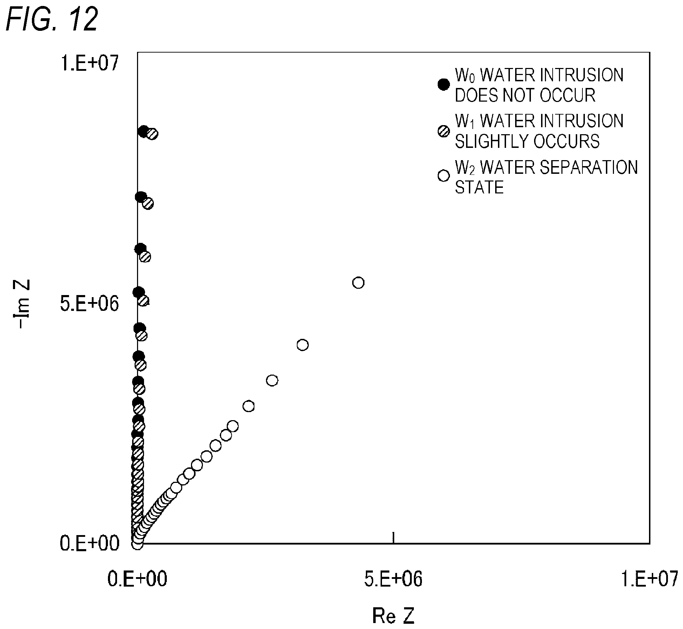

Next, a change in the output according to the degree of the intrusion of water into the lubricant will be described. FIG. 12 is a Nyquist diagram showing a state in which the output changes according to the degree of the intrusion of water into the lubricant. Here, three examples (intrusion ratios) are shown as water intrusion states, and the intrusion ratio is $W_0$ (= 0%) < $W_1$ < $W_2$. Further, it is assumed that the lubricant and water are separated at $W_2$.

As shown in FIG. 12, the larger the degree of the intrusion of water into the lubricant, the higher the level of the deviation from the case in which the intrusion of water does not occur. Therefore, by defining the tendency of the change in the output during such a frequency sweep in advance and comparing the tendency with the detection result, it is possible to estimate the ratio of intruded water to the lubricant. That is, the characteristics acquired when the frequency is swept may also change according to the intrusion ratio of water.

As described above, according to the present embodiment, it is possible to provide a method of detecting intrusion of water into a lubricant in a bearing device according to an electrical technique. According to the method of the present embodiment, it is also possible to estimate the degree of the intrusion of water into the lubricant. Further, the state diagnosis can be easily performed based on the above.

<Other Embodiments>

In the present invention, a program or an application for implementing functions of the one or more embodiments described above may be supplied to a system or a device using a network or a storage medium, and a process in which one or more processors in a computer of the system or the device read and execute the program may be implemented.

In addition, the process may be implemented by a circuit (for example, an application specific integrated circuit (ASIC) or a field programmable gate array (FPGA)) that implements one or more functions.

As described above, the present invention is not limited to the above embodiments, and combinations of the respective configurations of the embodiments and changes and modifications made by those skilled in the art based on the descriptions in the description and the well-known technique are intended by the present invention and are thus also included within the scope of the present invention to be protected.

As described above, the following matters are disclosed in the present description.

(1) A water intrusion detection method for detecting intrusion of water into a lubricant in a device in which a plurality of portions are lubricated using the lubricant, the method comprising:

a measurement step of measuring an impedance of an electric circuit including the plurality of portions by applying an AC voltage to the electric circuit while sweeping a frequency; and
a detection step of detecting the intrusion of water into the lubricant by comparing characteristics indicated by a real part and an imaginary part of an impedance measured in a state in which water is not mixed into the lubricant with characteristics indicated by a real part and an imaginary part of the impedance measured in the measurement step.
With this configuration, it is possible to provide the method of detecting intrusion of water into a lubricant in a bearing device according to an electrical technique. It is also possible to estimate the degree of the intrusion of water into the lubricant. Further, the state diagnosis can be easily performed based on the above.

(2) In the water intrusion detection method according to (1), in the detection step, a shape in a Nyquist diagram is used as the characteristics indicated by the real part and the imaginary part of the impedance.
With this configuration, it is possible to detect the intrusion of water into the lubricant by corresponding to the shape in the Nyquist diagram indicated by the real part and the imaginary part of the impedance.
(3) In the water intrusion detection method according to (1) or (2), the detection step further detects contact at the plurality of portions by comparing the characteristics indicated by the real part and the imaginary part of the impedance measured in the state in which water is not mixed into the lubricant with the characteristics indicated by the real part and the imaginary part of the impedance measured in the measurement step.
With this configuration, it is possible to identify and detect the contact between the components in addition to the presence or absence of the intrusion of water into the lubricant.
(4) The water intrusion detection method according to any one of (1) to (3) further includes a diagnosis step of diagnosing a state of the device by using a detection result of the detection step.
With this configuration, it is possible to diagnose an abnormal state of the device according to the detection result of the intrusion of water into the lubricant.
(5) In the water intrusion detection method according to any one of (1) to (4), the device is a rolling device.
With this configuration, it is possible to detect the intrusion of water into the lubricant with respect to the rolling device.
(6) In the water intrusion detection method according to any one of (1) to (4), the device is a bearing device, and the plurality of portions include an outer member, an inner member, and a rolling element.
With this configuration, it is possible to detect the intrusion of water into the lubricant with respect to the bearing device.
(7) A water intrusion detection device for detecting intrusion of water into a lubricant in a device in which a plurality of portions are lubricated using the lubricant, includes:

a measurement unit configured to measure an impedance of an electric circuit including the plurality of portions by applying an AC voltage to the electric circuit while sweeping a frequency; and
a detection unit configured to detect the intrusion of water into the lubricant by comparing characteristics indicated by a real part and an imaginary part of an impedance measured in a state in which water is not mixed into the lubricant with characteristics indicated by a real part and an imaginary part of the impedance measured by the measurement unit.
With this configuration, it is possible to provide the method of detecting intrusion of water into a lubricant in a bearing device according to an electrical technique. It is also possible to estimate the degree of the intrusion of water into the lubricant. Further, the state diagnosis can be easily performed based on the above.

(8) A program for causing a computer to execute:

a measurement step of measuring, in a device in which a plurality of portions are lubricated using a lubricant, an impedance of an electric circuit including the plurality of portions by applying an AC voltage to the electric circuit while sweeping a frequency; and
a detection step of detecting the intrusion of water into the lubricant by comparing characteristics indicated by a real part and an imaginary part of an impedance measured in a state in which water is not mixed into the lubricant with characteristics indicated by a real part and an imaginary part of the impedance measured in the measurement step.

With this configuration, it is possible to provide the method of detecting intrusion of water into a lubricant in a bearing device according to an electrical technique. It is also possible to estimate the degree of the intrusion of water into the lubricant. Further, the state diagnosis can be easily performed based on the above.

Although various embodiments have been described above, it is needless to mention that the present invention is not limited to such embodiments. It is apparent for those skilled in the art to which the present disclosure belongs that various modified examples or corrected examples are conceivable within the scope recited in the claims, and it is understood that the above falls within the technical scope of the present invention. In addition, the components described in the above embodiments may be combined in any manner without departing from the spirit of the invention.

The present application is based on a Japanese patent application (No. 2022-114947) filed on July 19, 2022, contents of which are incorporated herein by reference.

REFERENCE SIGNS LIST

1 diagnostic device
2 bearing device
3 outer ring (outer member)
4 inner ring (inner member)
5 rolling element
6 seal
7 rotation shaft
8 LCR meter
9 rotation connector
10 motor

**Claims**

1.  A water intrusion detection method for detecting intrusion of water into a lubricant in a device in which a plurality of portions are lubricated using the lubricant, the method comprising:

    a measurement step of measuring an impedance of an electric circuit including the plurality of portions by applying an AC voltage to the electric circuit while sweeping a frequency; and
    a detection step of detecting the intrusion of water into the lubricant by comparing characteristics indicated by a real part and an imaginary part of an impedance measured in a state in which water is not mixed into the lubricant with characteristics indicated by a real part and an imaginary part of the impedance measured in the measurement step.

2.  The water intrusion detection method according to claim 1, wherein
    in the detection step, a shape in a Nyquist diagram is used as the characteristics indicated by the real part and the imaginary part of the impedance.

3.  The water intrusion detection method according to claim 1, wherein
    the detection step further detects contact at the plurality of portions by comparing the characteristics indicated by the real part and the imaginary part of the impedance measured in the state in which water is not mixed into the lubricant with the characteristics indicated by the real part and the imaginary part of the impedance measured in the measurement step.

4. The water intrusion detection method according to claim 1, further comprising:
   a diagnosis step of diagnosing a state of the device by using a detection result of the detection step.

5. The water intrusion detection method according to any one of claims 1 to 4, wherein
   the device is a rolling device.

6. The water intrusion detection method according to any one of claims 1 to 4, wherein

   the device is a bearing device, and
   the plurality of portions include an outer member, an inner member, and a rolling element.

7. A water intrusion detection device for detecting intrusion of water into a lubricant in a device in which a plurality of portions are lubricated using the lubricant, the device comprising:

   a measurement unit configured to measure an impedance of an electric circuit including the plurality of portions by applying an AC voltage to the electric circuit while sweeping a frequency; and
   a detection unit configured to detect the intrusion of water into the lubricant by comparing characteristics indicated by a real part and an imaginary part of an impedance measured in a state in which water is not mixed into the lubricant with characteristics indicated by a real part and an imaginary part of the impedance measured by the measurement unit.

8. A program for causing a computer to execute:

   a measurement step of measuring, in a device in which a plurality of portions are lubricated using a lubricant, an impedance of an electric circuit including the plurality of portions by applying an AC voltage to the electric circuit while sweeping a frequency; and
   a detection step of detecting the intrusion of water into the lubricant by comparing characteristics indicated by a real part and an imaginary part of an impedance measured in a state in which water is not mixed into the lubricant with characteristics indicated by a real part and an imaginary part of the impedance measured in the measurement step.

FIG. 1

## FIG. 2

## FIG. 3

FIG. 4

OUTER
RING-ROLLING
ELEMENT

E2

$R_1$  $C_1$  $R_2$  $C_2$

INNER
RING-ROLLING
ELEMENT

E1

$R_1$  $C_1$  $R_2$  $C_2$

*FIG. 5A*

*FIG. 5B*

FIG. 6A

FIG. 6B

*FIG. 7A*

*FIG. 7B*

*FIG. 8*

```
                    ┌─────────────┐
                    │    START    │
                    └──────┬──────┘
                           │
                           ▼
     ┌──────────────────────────────────────────┐
     │   START ROTATION OF ROTATION SHAFT        │───S801
     └──────────────────┬───────────────────────┘
                        │
                        ▼
     ┌──────────────────────────────────────────┐
     │       INSTRUCT INPUT OF ω AND V           │───S802
     └──────────────────┬───────────────────────┘
                        │
                        ▼
     ┌──────────────────────────────────────────┐
     │          ACQUIRE IMPEDANCE Z              │───S803
     └──────────────────┬───────────────────────┘
                        │
                        ▼
     ┌──────────────────────────────────────────┐
     │  ESTIMATE CHANGE IN EQUIVALENT CIRCUIT    │
     │   BASED ON PARAMETERS OF REAL PART AND    │───S804
     │    IMAGINARY PART OF IMPEDANCE            │
     └──────────────────┬───────────────────────┘
                        │
                        ▼
     ┌──────────────────────────────────────────┐
     │    DETECT MIXING OF WATER BASED           │
     │      ON ESTIMATION RESULT                 │───S805
     └──────────────────┬───────────────────────┘
                        │
                        ▼
     ┌──────────────────────────────────────────┐
     │       NOTIFY DETECTION RESULT             │───S806
     └──────────────────┬───────────────────────┘
                        │
                        ▼
                 ┌─────────────┐
                 │     END     │
                 └─────────────┘
```

*FIG. 9*

EP 4 560 160 A1

FIG. 10

EP 4 560 160 A1

*FIG. 11*

○ WATER IS PRESENT

● METAL CONTACT OCCURS

Z REAL PART

- Z IMAGINARY PART

FIG. 12

## INTERNATIONAL SEARCH REPORT

| International application No. |
| --- |
| **PCT/JP2023/026157** |

| A. | CLASSIFICATION OF SUBJECT MATTER |
| --- | --- |

*F16C 19/04*(2006.01)i; *F16C 19/52*(2006.01)i; *F16C 41/00*(2006.01)i; *G01M 13/04*(2019.01)i
FI:    G01M13/04; F16C19/04; F16C19/52; F16C41/00

According to International Patent Classification (IPC) or to both national classification and IPC

| B. | FIELDS SEARCHED |
| --- | --- |

Minimum documentation searched (classification system followed by classification symbols)

G01M13/0-13/045, G01N27/00-27/10, G01N27/14-27/24, G01B7/00-7/34, F16C19/00-19/56

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

| C. | DOCUMENTS CONSIDERED TO BE RELEVANT | |
| --- | --- | --- |

| Category* | Citation of document, with indication, where appropriate, of the relevant passages | Relevant to claim No. |
| --- | --- | --- |
| Y | WO 2014/094812 A1 (AKTIEBOLAGET SKF) 26 June 2014 (2014-06-26)<br>paragraphs [0005], [0010], [0011], [0040], claims 4, 6, fig. 3 | 1-8 |
| Y | JP 7057868 B1 (NSK LTD.) 20 April 2022 (2022-04-20)<br>claims 1, 5, paragraphs [0014], [0017], fig. 1 | 1-8 |
| Y | JP 2007-502411 A (EXXONMOBIL RESEARCH AND ENGINEERING COMPANY) 08 February 2007 (2007-02-08)<br>paragraphs [0020], [0038], [0042] | 2 |
| Y | JP 2002-131188 A (SANKYO SEIKI MFG CO LTD) 09 May 2002 (2002-05-09)<br>paragraph [0013] | 3 |
| A | US 2018/0238851 A1 (AKTIEBOLAGET SKF) 23 August 2018 (2018-08-23)<br>entire text, all drawings | 1-8 |
| A | JP 6993652 B1 (TOKYO GAS CO LTD) 13 January 2022 (2022-01-13)<br>entire text, all drawings | 1-8 |

☐ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **18 August 2023** | **29 August 2023** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT
### Information on patent family members

International application No.

**PCT/JP2023/026157**

| Patent document cited in search report | | | Publication date (day/month/year) | Patent family member(s) | | | Publication date (day/month/year) |
|---|---|---|---|---|---|---|---|
| WO | 2014/094812 | A1 | 26 June 2014 | EP | 2932248 | A1 | |
| JP | 7057868 | B1 | 20 April 2022 | WO | 2022/071164 | A1 | |
| | | | | TW | 202217163 | A | |
| JP | 2007-502411 | A | 08 February 2007 | US | 2005/0035755 | A1 | |
| | | | | paragraphs [0039], [0056], [0061] | | | |
| | | | | WO | 2005/019794 | A2 | |
| | | | | EP | 1668335 | A1 | |
| | | | | CA | 2535003 | A | |
| | | | | AU | 2004267368 | A | |
| JP | 2002-131188 | A | 09 May 2002 | US | 2002/0083779 | A1 | |
| | | | | CN | 1350168 | A | |
| | | | | SG | 104946 | A | |
| US | 2018/0238851 | A1 | 23 August 2018 | GB | 2559791 | A | |
| | | | | GB | 201702678 | D0 | |
| | | | | DE | 102018201562 | A | |
| | | | | CN | 108459057 | A | |
| JP | 6993652 | B1 | 13 January 2022 | (Family: none) | | | |

Form PCT/ISA/210 (patent family annex) (January 2015)

24

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2019211317 A